# EUROPEAN PATENT APPLICATION

(11) **EP 1 982 714 A1**
(43) Date of publication of application: **22.10.2008**
(21) Application number: 07384022.5
(22) Date of filing: 16.04.2007
(51) Int. Cl.: A61K 31/4162, C07D 491/04, A61P 25/00, A61P 23/00

(54) **Pyrano-pyrazole-amines**

(71) Applicant: Laboratorios del Dr. Esteve S.A., 08041 Barcelona (ES)
(72) Inventor: Wünsch, Bernard Prof. Dr, 48161 Münster (DE); Schepmann, Dirk Dr., 48147 Münster (DE); Schläger, Torsten, 48151 Münster (DE); Zamanillo-Castenedo, Daniel Dr., 08041 Barcelona (ES)
(74) Representative: Peters, Hajo

(57) **Abstract**

The present invention relates to compounds of formula (I) having pharmacological activity towards the sigma (σ) receptor, and more particularly to Pyrano-pyrazol-amine, to processes of preparation of such compounds, to pharmaceutical compositions comprising them, and to their use in therapy and prophylaxis, in particular for the treatment of psychosis.

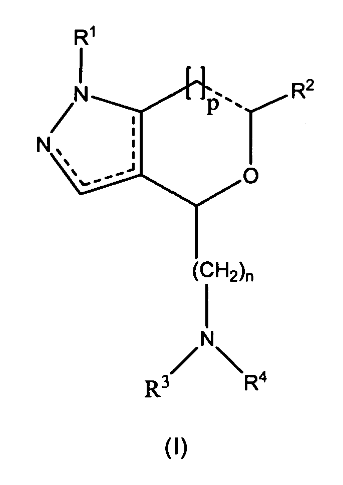

## Description

### FIELD OF THE INVENTION

The present invention relates to compounds having pharmacological activity towards the sigma (σ) receptor, and more particularly to some pyrano-pyrazole-amines, to processes of preparation of such compounds, to pharmaceutical compositions comprising them, and to their use in therapy and prophylaxis, in particular for the treatment of psychosis.

### BACKGROUND OF THE INVENTION

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

There is still a need to find compounds that have pharmacological activity towards the sigma receptor, being both effective and selective, and having good "drugability" properties, i.e. good pharmaceutical properties related to administration, distribution, metabolism and excretion.

The closest technique known comprises benzimidazoles of WO2003035065 for the inhibition of kinases. Tetrahydro-pyranopyrazole compounds displaying cannabinoid modulating activity are disclosed in W02007001939 and FR2875230. None of the them presents spiro-pyrano-pyrazole variants or analogues.

Spiropiperidines are known as potent ligands to sigma receptors (Maier et al, J Med Chem, 2002, 45, 438-448 and Maier et al, J Med Chem, 2002, 45, 4923-4930). However, such spiropiperidines show benzofuran and benzopyran rings.

### SUMMARY OF THE INVENTION

We have now found a family of structurally distinct spiro[benzopyran] or spiro[benzofuran] Derivatives which are particularly selective inhibitors of the sigma receptor.

The invention is directed to compounds of general formula (I), 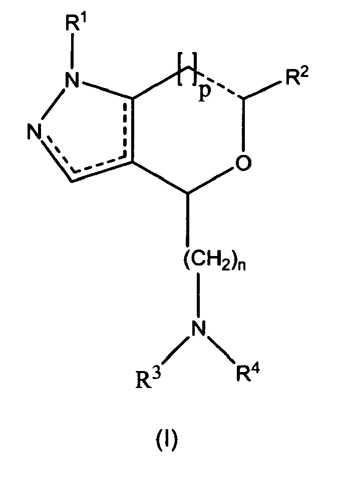 wherein
n is selected from 0,1, 2 or 3;
p is selected from 0 or 1;
the dotted line ......... is either a double or a single bond;
if p is 1, the dotted line ......... is either a double or a single bond;
if p is o, the dotted line ......... is a single bond;
R¹ is selected from hydrogen; at least mono-substituted, linear or branched C₁₋₆-aliphatic group; optionally at least monosubstituted aryl; optionally at least mono-subtituted alkyl-aryl;
R² is selected from hydrogen; an optionally at least monosubstituted, linear or branched C₁₋₆-aliphatic group; O-R with R being H or an optionally at least monosubstituted, linear or branched C₁₋₆-aliphatic group;
R³ and R⁴ independently of one another are selected from hydrogen; an optionally at least monosubstituted, linear or branched C₁₋₁₈-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;
   or
R³ and R⁴ together with the connecting nitrogen form an optionally at least mono-substituted heterocyclyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In the context of this invention aliphatic group or aliphatic radical includes alkyl, alkenyl and alkinyl.

In the context of this invention, alkyl radical or group is understood as meaning saturated, linear or branched hydrocarbons, which can be unsubstituted or mono- or polysubstituted. Alkenyl and alkinyl groups, on the other hand include groups like e.g. -CH=CH-CH₃ or -C≡C-CH₃, while the saturated alkyl encompasses e.g. -CH₃ and - CH₂-CH₃. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-. C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

In the context of this invention cycloalkyl radical or group is understood as meaning saturated and unsaturated (but not aromatic) cyclic hydrocarbons (without a heteroatom in the ring), which can be unsubstituted or mono- or polysubstituted. Furthermore, C₃₋₄-cycloalkyl represents C3- or C4-cycloalkyl, C₃₋₅-cycloalkyl represents C3-, C4- or C5-cycloalkyl, C₃₋₆-cycloalkyl represents C3-, C4-, C5- or C6-cycloalkyl, C₃₋₇cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. However, mono- or polyunsaturated, preferably monounsaturated, cycloalkyls also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

In the context of this invention alkyl-cycloalkyl is understood as meaning a cycloalkyl group (see above) being connected to another atom through a C₁₋₆-alkyl group (see above), whereas the C₁₋₆-alkyl-group is always saturated and unsubstituted, and linear or branched.

In connection with alkyl or aliphatic group - unless defined otherwise - the term substituted in the context of this invention is understood as meaning replacement of at least one hydrogen radical by F, Cl, Br, I, NH₂, SH or OH, "polysubstituted" (more than once substituted) radicals being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂. "Optionally at least monosubstituted" means either "monosubstituted", "polysubstituted" or - if the option is not fulfilled - "unsubstituted".

The term (CH₂)₃₋₆ is to be understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc.

An aryl radical or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention alkyl-aryl is understood as meaning an aryl group (see above) being connected to another atom through a C₁₋₆-alkyl-group (see above), whereas the C₁₋₆-alkyl-group is always saturated and unsubstituted, and linear or branched.

A heterocyclyl radical or group is understood as meaning heterocyclic ring systems, saturated or unsaturated ring which contains one or more heteroatoms from the group consisting of nitrogen, oxygen and/or sulfur in the ring and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In the context of this invention alkyl-heterocylyl is understood as meaning a heterocyclyl group (see above) being connected to another atom through a C₁₋₆-alkyl group (see above), whereas the C₁₋₆-alkyl-group is always saturated and unsubstituted, and linear or branched.

In connection with aryl or alkyl-aryl, cycloalkyl or alkyl-cycloalkyl, heterocyclyl or alkyl-heterocyclyl, substituted is understood - unless defined otherwise - as meaning substitution of the ring-system of the aryl or alkyl-aryl, cycloalkyl or alkyl-cycloalkyl; heterocyclyl or alkyl-heterocyclyl by OH, SH, =O, halogen (F, Cl, Br, I), CN, NO₂, COOH; NRₓR_{y}, with Rₓ and R_{y} independently being either H or a saturated or unsaturated, linear or branched, substituted or unsubstituted C₁₋₆-alkyl; a saturated or unsaturated, linear or branched, substituted or unsubstituted C₁₋₆-alkyl; a saturated or unsaturated, linear or branched, substituted or unsubstituted -O-C₁₋₆₋alkyl (alkoxy); a saturated or unsaturated, linear or branched, substituted or unsubstituted -S-C₁₋₆-alkyl; a saturated or unsaturated, linear or branched, substituted or unsubstituted - C(O)-C₁₋₆₋alkyl-group; a saturated or unsaturated, linear or branched, substituted or unsubstituted -C(O)-O-C₁₋₆₋alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl. "Optionally at least monosubstituted" means either "monosubstituted", "polysubstituted" or - if the option is not fulfilled - "unsubstituted".

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids and in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Any compound that is a prodrug of a compound of formula (I) is within the scope of the invention. The term "prodrug" is used in its broadest sense and encompasses those Derivatives that are converted in vivo to the compounds of the invention. Such Derivatives would readily occur to those skilled in the art, and include, depending on the functional groups present in the molecule and without limitation, the following Derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).
Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

In a preferred embodiment of the compound according to the invention according to general formula I R¹ is selected from hydrogen; at least mono-substituted, linear or branched C₁₋₆-aliphatic group; optionally at least monosubstituted aryl; especially R¹ is selected from linear or branched C₁₋₄-alkyl; or optionally at least monosubstituted aryl; more preferably R¹ is selected from CH₃ or phenyl.

In another preferred embodiment of the compound according to the invention according to general formula I R² is selected from H, or OR with R being H or an optionally at least monosubstituted, linear or branched C₁₋₆-aliphatic group; especially R² is selected from H or OR with R being H or a linear or branched C₁₋₄-alkyl group, more preferably R² is selected from H, OH or OCH₃; most preferably R² is selected from H.

In another preferred embodiment of the compound according to the invention according to general formula I
R³ is selected from H; an optionally at least monosubstituted, linear or branched C₁₋₆-aliphatic group; an optionally at least monosubstituted alkyl-aryl; an optionally at least monosubstituted alkyl-heterocyclyl; or an optionally at least monosubstituted alkyl-cycloalkyl;
while R⁴ is selected from H; or an optionally at least monosubstituted, linear or branched C₁₋₆-aliphatic group;
   or
R³ and R⁴ together with the connecting nitrogen form an optionally at least mono-substituted heterocyclyl group.

In another preferred embodiment of the compound according to the invention according to general formula I n is selected from 1 or 2.

In another preferred embodiment of the compound according to the invention the compound is a compound according to general formula Ia 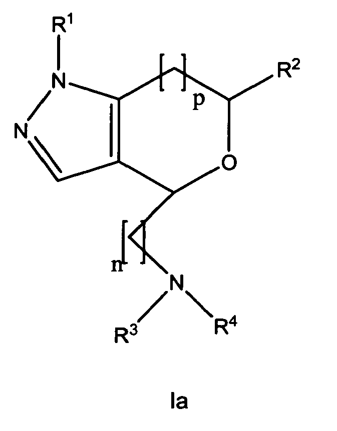 wherein
p is selected from 0 or 1;
n is selected from 1 or 2;
R¹ is selected from hydrogen; at least mono-substituted, linear or branched C₁₋₆-aliphatic group; optionally at least monosubstituted aryl; optionally at least mono-subtituted alkyl-aryl;
R² is selected from hydrogen; an optionally at least monosubstituted, linear or branched C₁₋₆-aliphatic group; or OR with R being H or an optionally at least monosubstituted, linear or branched C₁₋₆-aliphatic group;
R³ is selected from hydrogen; an optionally at least monosubstituted, linear or branched C₁₋₆-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;
while R⁴ is selected from hydrogen; an optionally at least monosubstituted, linear or branched C₁₋₆-aliphatic group;
   or
R³ and R⁴ together with the connecting nitrogen form an optionally at least mono-substituted heterocyclyl group.

In a preferred embodiment of the compound according to the invention according to general formula la R¹ is selected from hydrogen; at least mono-substituted, linear or branched C₁₋₆-aliphatic group; optionally at least monosubstituted aryl; especially R¹ is selected from linear or branched C₁₋₄-alkyl; or optionally at least monosubstituted aryl; more preferably R¹ is selected from CH₃ or phenyl.

In another preferred embodiment of the compound according to the invention according to general formula la R² is selected from H; OR with R being H or an optionally at least monosubstituted, linear or branched C₁₋₆-alkyl group; especially R² is selected from H; OH or a linear or branched OC₁₋₄-alkyl group; more preferably R² is selected from H, OH or OCH₃.

In another preferred embodiment of the compound according to the invention according to general formula la
R³ is selected from hydrogen; an optionally at least monosubstituted, linear or branched C₁₋₆-alkyl;
while R⁴ is selected from hydrogen; or an optionally at least monosubstituted, linear or branched C₁₋₆-alkyl group;
   or
R³ and R⁴ together with the connecting nitrogen form an optionally at least mono-substituted 5- or 6-membered saturated heterocyclyl group.

Another preferred embodiment of the compound according to the invention is a compound according to general formula Ia, 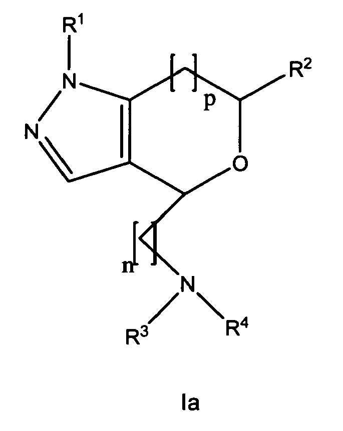 wherein
p is 1;
n is 1 or 2;
R¹ is selected from linear or branched C₁₋₄-alkyl; or optionally at least monosubstituted aryl;
R² is selected from H; OH or a linear or branched OC₁₋₄-alkyl group;
R³ is selected from hydrogen; an optionally at least monosubstituted, linear or branched C₁₋₆-alkyl;
while R⁴ is selected from hydrogen; or an optionally at least monosubstituted, linear or branched C₁₋₆-alkyl group;
   or
R³ and R⁴ together with the connecting nitrogen form an optionally at least mono-substituted 5- or 6-membered saturated heterocyclyl group.

Another preferred embodiment of the compound according to the invention is a copound according to general formula la 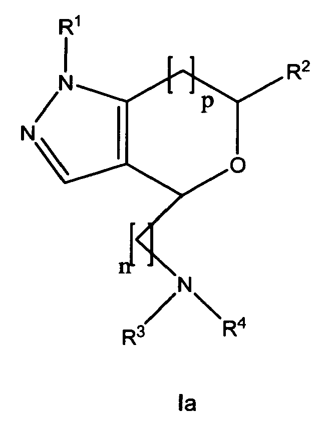 wherein
p is 1;
n is 1 or 2;
R¹ is selected from linear or branched C₁₋₄-alkyl; or optionally at least monosubstituted aryl;
R² is selected from H; OH or a linear or branched OC₁₋₄-alkyl group;
R³ is selected from hydrogen; an optionally at least monosubstituted, linear or branched C₁₋₆-alkyl;
while R⁴ is selected from hydrogen; or an optionally at least monosubstituted, linear or branched C₁₋₆-alkyl group;
   or
R³ and R⁴ together with the connecting nitrogen form an optionally at least mono-substituted 5- or 6-membered saturated heterocyclyl group, selected from: 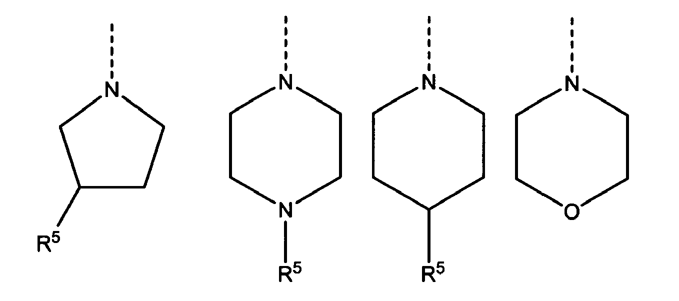
with R⁵ being selected from hydrogen; an optionally at least monosubstituted, linear or branched C₁₋₆-alkyl group; or an optionally at least monosubstituted aryl group.

In another preferred embodiment of the compound according to the invention according to general formula I the compound is selected from
- 1-Phenyl-4-(piperidin-1-ylmethyl)-1,4,6,7-tetrahydro-pyrano[4,3-c]pyrazole;
- 1-Phenyl-4-(2-piperidin-1-yl-ethyl)-1,4,6,7-tetrahydro-pyrano[4,3-c]pyrazole
- 1-Phenyl-4-(4-phenylpiperidin-1-ylmethyl)-1,4,6,7-tetrahydropyrano[4,3-c]pyrazole;
- 1-Phenyl-4-[2-(4-phenyl-piperidin-1-yl)-ethyl]-1,4,6,7-tetrahydro-pyrano[4,3-c]pyrazole
- 4-(Morpholin-4-ylmethyl)-1-phenyl-1,4,6, 7-tetrahydro-pyrano[4, 3-c]pyrazole;
- 4-(2-Morpholin-4-yl-ethyl)-1-phenyl-1,4,6,7-tetrahydro-pyrano[4.3-c]pyrazole
- 1-Phenyl-4-(4-phenylpiperazin-1-ylmethyl)-1,4,6,7-tetrahydropyrano[4,3-c]pyrazole;
- 1-Phenyl-4-[2-(4-phenyl-piperazin-1-yl)-ethyl]-1,4,6,7-tetrahydro-pyrano[4,3-c]pyrazole;
- 4-(4-Methylpiperazin-1-ylmethyl)-1-phenyl-1,4,6,7-tetrahydropyrano[4,3-c]pyrazole;
- 4-[2-(4-Methyl-piperazin-1-yl)-ethyl]-1-phenyl-1,4,6,7-tetrahydro-pyrano[4,3-c] pyrazole
- 1-Phenyl-4-(pyrrolidin-1-ylmethyl)-1,4,6,7-tetrahydro-pyrano[4,3-c]pyrazole;
- 1-Phenyl-4-(2-pyrrolidin-1-yl-ethyl)-1,4,6,7-tetrahydro-pyrano[4,3-c]pyrazole;
- N,N-Dimethyl-1-(1-phenyl-1,4,6,7-tetrahydropyrano[4,3-c]pyrazole-4-yl)methanamine; or
- Dimethyl-[2-(1-phenyl-1,4,6,7-tetrahydro-pyrano[4,3-c]pyrazol-4-yl)-ethyl]-amine;
   preferably
- 1-Phenyl-4-(piperidin-1-ylmethyl)-1,4,6,7-tetrahydro-pyrano[4,3-c]pyrazole;
- 1-Phenyl-4-(4-phenylpiperidin-1-ylmethyl)-1,4,6,7-tetrahydropyrano[4,3-c]pyrazole;
- 4-(Morpholin-4-ylmethyl)-1-phenyl-1,4,6,7-tetrahydro-pyrano[4,3-c]pyrazole;
- 1-Phenyl-4-(4-phenylpiperazin-1-ylmethyl)-1,4,6,7-tetrahydropyrano[4,3-c]pyrazole;
- 4-(4-Methylpiperazin-1-ylmethyl)-1-phenyl-1,4,6,7-tetrahydropyrano[4,3-c]pyrazole;
- 1-Phenyl-4-(pyrrolidin-1-ylmethyl)-1,4,6,7-tetrahydro-pyrano[4,3-c]pyrazole; or
- N,N-Dimethyl-1-(1-phenyl-1,4,6,7-tetrahydropyrano[4,3-c]pyrazole-4-yl)methanamine;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

The term "pharmacological tool" refers to the property of compounds of the invention through which they are particularly selective ligands for Sigma receptors which implies that compound of formula (I), described in this invention, can be used as a model for testing other compounds as sigma ligands, ex. a radiactive ligands being replaced, and can also be used for modeling physiological actions related to sigma receptors.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

In general the processes are described below in the experimental part. The starting materials are commercially available or can be prepared by conventional methods.

A preferred aspect of the invention is also a process for the production of a compound according to formula Ib, 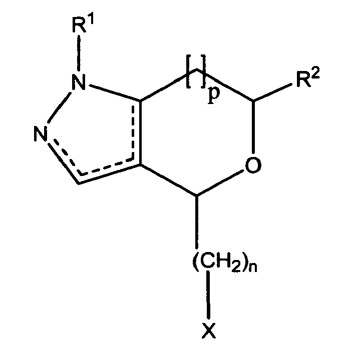 wherein R¹, R² n, and p are as defined above and X is a leaving group, preferably a halogen,
wherein a compound of formula III 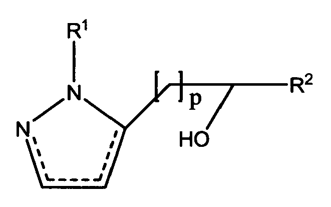 , wherein R¹, R² and p are as defined above, is reacted with a compound of general formula IV 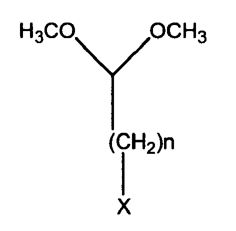 wherein n is as defined as above and X is a leaving group, preferably is a halogen, more preferably is Br or Cl;
to form a compound according to formula lb.

Another preferred aspect of the invention is a process for the production of a compound according to the invention, wherein a compound of formula Ib, 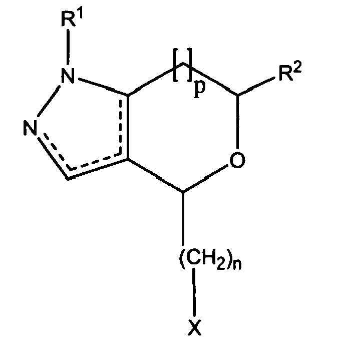 wherein R¹, R² n and p are as defined above and X is a leaving group, preferably a halogen, more preferably Br or CI; is reacted with R³R⁴NH, wherein R³ and R⁴ are as defined above.

The obtained reaction products may, if desired, be purified by conventional methods, such as crystallisation and chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

Another aspect of the invention refers to a pharmaceutical composition which comprises a compound according to the invention or a pharmaceutically acceptable salt, prodrug, isomer or solvate thereof, and a pharmaceutically acceptable carrier, adjuvant or vehicle. The present invention thus provides pharmaceutical compositions comprising a compound of this invention, or a pharmaceutically acceptable salt, derivative, prodrug or stereoisomers thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of the invention refers to the use of a compound according to the invention in the manufacture of a medicament.

Another aspect of the invention refers to the use of a compound according to the invention in the manufacture of a medicament for the treatment or prophylaxis of a sigma receptor mediated disease or condition. A preferred embodiment of this is this use wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer.

A preferred embodiment of this is this use wherein the disease is pain, especially neuropathic pain, inflammatory pain or other pain conditions, allodynia and/or hyperalgesia, especially mechanical allodynia.

Another aspect of the invention refers to the use of a compound according o the invention as pharmacological tool or as anxiolytic or immunosuppressant.

The term "pharmacological tool" refers to the property of compounds of the invention through which they are particularly selective ligands for Sigma receptors which implies that compound of formula I, described in this invention, can be used as a model for testing other compounds as Sigma ligands, ex. a radiactive ligands being replaced, and can also be used for modeling physiological actions related to Sigma receptors.

Another aspect of this invention relates to a method of treating or preventing a sigma receptor mediated disease which method comprises administering to a patient in need of such a treatment a therapeutically effective amount of a compound as above defined or a pharmaceutical composition thereof. Among the sigma mediated diseases that can be treated are diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, pain, especially neuropathic pain, inflammatory pain or other pain conditions, allodynia and/or hyperalgesia, especially mechanical allodynia, psychotic condition, schizophrenia; inflammation, autoimmune diseases or cancer; disorders of food ingestion, the regulation of appetite, for the reduction, increase or maintenance of body weight, for the prophylaxis and/or treatment of obesity, bulimia, anorexia, cachexia or type II diabetes, preferably type II diabetes caused by obesity. The compounds of the invention can also be employed as pharmacological tool or as anxiolytic or immunosuppressant.

The compounds of the invention may be synthesized following Reaction Scheme A set out below: 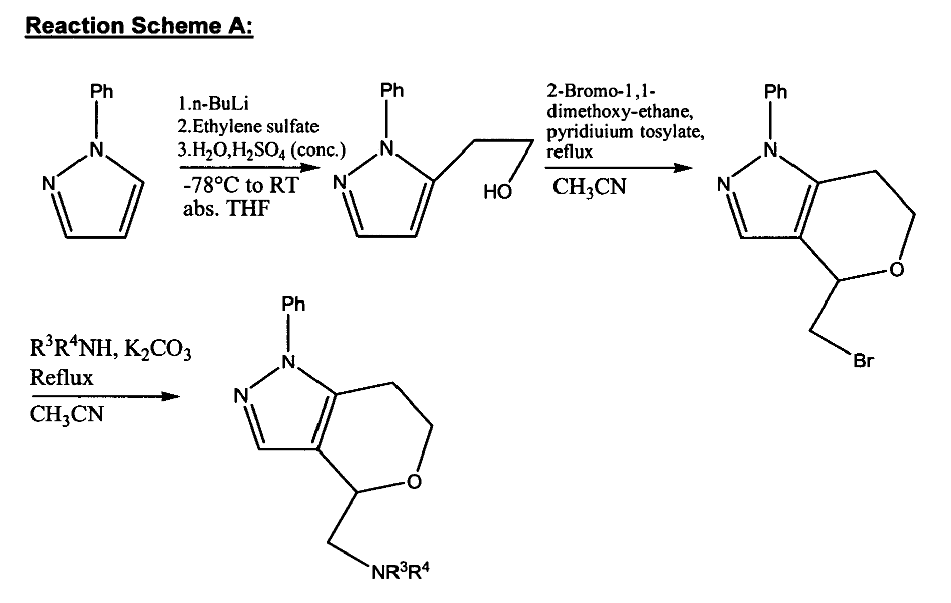 The present invention is illustrated below with the aid of examples. These illustrations are given solely by way of example and do not limit the general spirit of the present invention.

### EXAMPLES:

### General Experimental Part (Methods and Equipment of the synthesis and

### analysis

All solvents used for synthesis were p. a. quality.

The separation of mixtures of substances using thin-layer chromatography was done on glass plates layered by silica gel. Analysis of the plates was done after treatment with iodine gas or incubation with Dragendorffs reagent under UV light.

As a rule synthesized products were purified using flash-chromatography.

Melting points were measured using capillaries. As sometimes the products were mixtures of diastereoisomers, themelting point: had to be expressed as a range.

IR-spectra were measured using the FT-IR-480 Plus Fourier Transform Spectrometer with ATR (Fa. Jasco). All substances were either measured directly as solids or in oil.

NMR-Spectra were measured using Mercury-400BB (Fa. Varian) at a temperature of 21°C. δ, measured in ppm, is based on the signal TMS measured in comparison to the residue signal (CHCl₃) of the solvent (CDCl₃):
¹H-NMR-Spectroscopy:
*δ* (TMS) = *δ* (CHCl₃) - 7.26
¹³C-NMR-Spectroscopy:
δ (TMS) *= δ* (CHCl₃) - 77.0

Mass-spectra (MS) were measured using the GCQ Finnigan MAT (Fa. Finnigan) with Xcalibur Version 1.1. The method of ionisation is shown in brackets: EI = electronic ionisation (70 eV); CI = Chemical ionisation (Isobutane or NH₃, 170 eV).

Elementary analysis was done with VarioEL (Fa. Elementar).

Before using HPLC the maximum absorption of the substances was measured using the UV/Vis-Spectra done with a 50Bio Cary Spektrophotometer (Fa. Varian).

For determination of the purity and for the separation of the diastereomers a HPLC Hitachi L6200A Intelligent Pump with a UV-Detector (Merck) was used.

Synthesis was done in the synthetic microwave Discover (Fa. CEM).

Some reactions were done using protective gas.

Reactions at -78°C were done in an acetone bath in a Dewar.

### Example A: 2-(1-Phenylpyrazole-5-yl)-ethanol

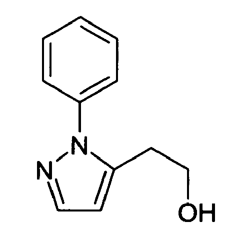

### Experimental Procedure:

Under N₂-atmosphere phenyl-pyrazole (1.0 g, 6.94 mmol) was dissolved in abs. THF (70 mL) and cooled to -78 °C. Following that n-buthyl lithium in hexane (1.6 M, 4.3 mL, 6.94 mmol) was added dropwise and thereafter the mixture was stirred for 2 h at -78 °C. Following that ethylene sulfate (1.03 g, 8.32 mmol), dissolved in abs. THF (10 mL) was slowly added. After a further hour of stirring at -78 °C the reaction mixture was heated to room temperature and stirred for a further 71 h. After addition of water (30 mL) and H₂SO_{4 conc}. (5 mL) it was heated for 40 h under reflux and strong stirring, before it was neutralized with NaOH (5 N, 60 mL) and extracted 3 times with CH₂Cl₂-The pooled organic phases were dried over K₂CO₃, filtered and the solvent removed in vacuum. The crude product (1.31 g) was prified using flash-chromatography (Ø=6 cm, h = 15 cm, n-hexane:ethyl acetate = 1:1, 40 mL, R_{f} = 0.13).
Slightly yellow oil, Yield: 981 mg (75%)
C₁₁H₁₂N₂O (188.3)

| | C | H | N |
|---|---|---|---|
| Calc. | 70.2 | 6.43 | 14.9 |
| Found | 70.2 | 6.55 | 14.9 |

**MS** (EI): m/z (rel. Int.) = 189 [MH⁺, 42], 188 [M⁺, 52], 158 [MH - CH₂OH, 41], 157 [M - CH₂OH, 100], 77 [Phenyl, 18].
**IR** (neat): *ṽ* (cm⁻¹) = 3328 (O-H), 3067 (C-H _{aromat.}), 2930, 2876 (C-H _{aliphat.}), 1598, 1534, 1501 (C=C), 764, 695 (C-H).
**¹H-NMR** (CDCl₃): δ (ppm) = 2.11 (s breit, 1H, CH₂CH₂O*H*), 2.91 (t, J = 6.5 Hz, 2H, C*H*₂CH₂OH), 3.79 (t, J = 6.7 Hz, 2H, CH₂C*H*₂OH), 6.29 (d, J = 1.6 Hz, 1 H, Pyrazol-4-C*H*), 7.38 - 7.48 (m, 5H, Phenyl-C*H*), 7.60 (d, J = 1.6 Hz, 1H, Pyrazol-3-C*H*).
**¹³C-NMR** (CDCl₃): δ (ppm) = 29.7 (1C, ArCH₂CH₂OH), 61.4 (1C, ArCH₂CH₂OH), 106.1 (1C, Pyrazol-4-CH), 125.9 (2C, Phenyl-CH, ortho), 128.4 (1C, Phenyl-CH, para), 129.4 (2C, Phenyl-CH, meta), 139.7 (1C, Phenyl-C, quartär), 140.2 (1C, Pyrazol-3-CH), 140.5 (1C, Pyrazol-5-C).

### Example B: 4-(Brommethyl)-1-phenyl-1,4,6,7-tetrahydropyrano-[4,3-c]pyrazole

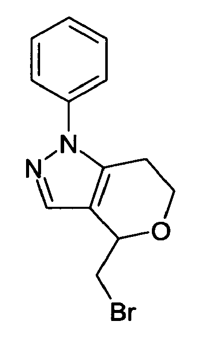

### Experimental procedure:

To a solution of example A (1.0 g, 5.31 mmol) in acetonitrile (35 mL) were added consecutively bromo acetaldehyd dimethylacetal (2-Bromo-1,1-dimethoxy-ethane) (941.9 µL, 7.97 mmol) und pyridinium tosylate (6.7 g, 26.6 mmol). The reaction mixture was stirred for 74 h under reflux. After removal of the solvent in vacuum the residue was dissolved in ethyl acetate and it was acidified with HCl (0.5 N) and extracted with ethyl acetate. The pooled organic phases were dried over K₂CO₃, filtered and the solvent removed in vacuum. The crude product (945 mg) was purified using flash-chromatography (∅ = 6 cm, h = 18 cm, n-hexane:ethylacetate 8:2, 40 mL, R_{f} = 0.29).
Slightly yellow solid, melting point 110°C, Yield: 529 mg (34 %).
C₁₃H₁₃BrN₂O (293.2)
**MS** (ESI): m/z (rel. Int.) = 293 [⁷⁹Br-M⁺, 45], 295 [⁸¹Br-M⁺, 37], 316 [⁷⁹Br-M + Na⁺, 53],
318 [⁸¹Br-M + Na⁺, 47], 609 [2 x ⁷⁹Br-M + Na⁺, 100].
**IR** (neat): *ṽ* (cm⁻¹) = 3049 (C-H _{aromat.}), 2977, 2935 (C-H _{aliphat.}), 2855 (C-H), 1596, 1503 (C=C), 1089, 1069 (C-O), 763, 964 (C-H).
**¹H-NMR** (CDCl₃): δ (ppm) = 2.68 - 2.77 (m, 1H, OCH₂C*H*₂Ar), 2.99 - 3.09 (m, 1H, OCH₂C*H*₂Ar), 3.57 (dd, J = 11.0/7.4 Hz, 1H, CHC*H*₂Br), 3.70 (dd, J = 10.8/4.1 Hz, 1H, CHC*H*₂Br), 3.76 (ddd, J = 11.5/9.6/3.7 Hz, 1 H, OC*H*₂CH₂Ar), 4.23 (ddd, J = 11.4/5.4/3.0 Hz, 1H, OC*H*₂CH₂Ar), 4.98 (dd, J = 7.2/4.1 Hz, 1H, C*H*CH₂Br), 7.37 (t, J = 7.0 Hz, 1H, Phenyl-*C*H, para), 7.42 - 7.53 (m, 4H, Phenyl-*C*H), 7.60 (s, 1H, Pyrazol-3-CH).

### Example 1: 1-Phenyl-4-(piperidin-1-ylmethyl)-1.4.6.7-tetrahydro-pyrano[4.3-c]pyrazole

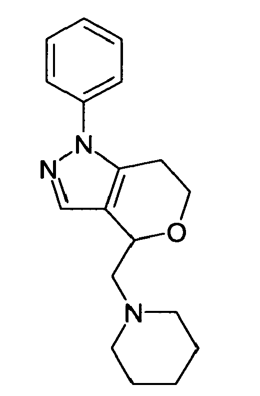

### Experimental Procedure:

To a solution of example B (80 mg, 0.27 mmol) in acetonitrile (5 mL) were added consecutively K₂CO₃ (301.7 mg, 2.18 mmol) and piperidin (80.9 µL, 0.81 mmol). This reaction mixture was stirred for 42 h under reflux, before K₂CO₃ was filtered off and the solvent was removed in vacuum. After the crude product (88.5 mg) was prurified using flash-chromatography (Ø = 3 cm, h = 18 cm, n-hexane: ethylacetate 5:5 + 1% N,N-dimethylethylamine, 20 mL, R_{f} = 0.11), the product was isolated.
Colourless solid, melting point: 91 °C, yield: 47 mg (58%).
C₁₈H₂₃N₃O (297.4)

| | C | H | N |
|---|---|---|---|
| Calc. | 72.7 | 7.80 | 14.1 |
| Found | 72.5 | 7.80 | 13.8 |

**MS** (ESI): m/z (rel. Int.) = 298 [MH⁺ 100], 617 [2x M + Na⁺, 15].
**IR** (neat): *v̅̃̅* (cm⁻¹) = 3059 (C-H _{aromat.}), 2930 (C-H _{aliphat.}), 2850 (C-H), 1599, 1504 (C=C), 1091 (C-O), 758, 693 (C-H).
**¹H-NMR** (CDCl₃): δ (ppm) = 1.38 - 1.45 (m, 2H, Piperidin-4-C*H₂*), 1.52 - 1.63 (m, 4H, Piperidin-3,5-C*H₂*), 2.40 - 2.69 (m, 7H, Piperidin-2,6-C*H₂* (4H), OCH₂C*H*₂Ar (1H), CHC*H₂*Piperidin (2H)), 2.93 - 3.03 (m, 1 H, OCH₂C*H₂*Ar), 3.61 (ddd, J = 11.3/10.2/3.9 Hz, 1H, OC*H*₂CH₂Ar), 4.97 (ddd, J = 11.5/5.7/2.2 Hz, 1 H, OC*H*₂CH₂Ar), 4.78 - 4.84 (m, 1H, C*H*CH₂Piperidin), 7.25 (t, J = 7.0 Hz, 1H, Phenyl-C*H*), 7.35 - 7.48 (m, 4H, Phenyl-C*H*), 7.51 (s, 1H, Pyrazol-3-C*H*).
**¹³C-NMR** (CDCl₃): δ (ppm) = 24.5 (1C, Piperidin-4'-CH₂), 25.1 (1C, OCH₂*C*H₂Ar), 26.1 (2C, Piperidin-3',5'-CH₂), 55.5 (2C, Piperidin-2',6'-CH₂), 63.6 (1C, OCH₂CH₂Ar), 64.5 (1C, CHCH₂Piperidin), 71.2 (1C, CHCH₂Piperidin), 77.4 (1C, Pyrazol-4-C), 122.8 (2C, Phenyl-CH, ortho), 127.2 (1C, Phenyl-CH, para), 129.5 (2C, Phenyl-CH, meta), 136.0 (1C, Phenyl-C, quartär), 136.5 (1C, Pyrazol-3-CH), 139.8 (1C, Pyrazol-5-C).

### Example 2: 1-Phenyl-4-(4-phenylpiperidin-1-ylmethyl)-1,4,6,7-tetrahydropyrano[4,3-c]pyrazole

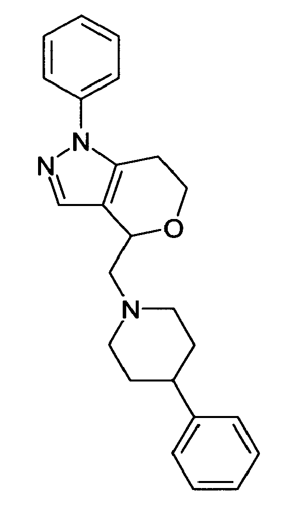

### Experimental Procedure:

To a solution of example B (80 mg, 0.27 mmol) in acetonitril (5 mL) were added consecutively K₂CO₃ (301.7 mg, 2.18 mmol) und 4-phenylpiperidin (132.0 mg, 0.82 mmol). This reaction mixture was stirred for 25 h unter reflux, before K₂CO₃ was filtered off and the solvent removed in vacuum. After the crude product (190 mg) was purified using flash-chromatography (0 = 3 cm, h = 20 cm, n-hexane: ethylacetate 5:5 + 1% N,N-dimethylethylamine, 20 mL, R_{f} = 0.25), the product was isolated.
Colourless resin, Yield 41 mg (41%).
C₂₄H₂₇N₃O (373.5)

| | C | H | N |
|---|---|---|---|
| Calc. | 77.2 | 7.29 | 11.3 |
| Found | 77.0 | 7.34 | 11.1 |

**MS** (ESI): m/z (rel. Int.) = 374 [MH⁺, 100], 769 [2 x M + Na⁺, 47].
**IR** (neat): *ṽ* (cm⁻¹) = 3058, 3026 (C-H _{aromat.}), 2932 (C-H _{aliphat.}), 2847 (C-H), 1598, 1504 (C=C), 1091 (C-O).
**¹H-NMR** (CDCl₃): δ (ppm) = 1.82 - 1.98 (m, 4H, Phenylpiperidin-3',5'-C*H₂*), 2.19 - 2.30 (m, 2H, Phenylpiperidin-2',6'-C*H*₂), 2.50 - 2.59 (m, 1H, Phenylpiperidin-4'-C*H*), 2.65 - 2.73 (m, 2H, OCH₂C*H*₂Ar (1H), CHC*H*₂Phenylpiperidin (1H)), 2.81 (dd, J = 13.3/8.2 Hz, 1H, CHC*H*₂-Phenylpiperidin), 3.02 - 3.11 (m, 1 H, OCH₂C*H*₂Ar), 3.22 (t breit, J = 10.0 Hz, 2H, Phenylpiperidin-2',6'-C*H*₂), 3.70 (ddd, J = 11.3/10.2/3.9 Hz, 1H, OC*H*₂CH₂Ar), 4.27 (ddd, J = 11.5/5.7/2.2 Hz, 1H, OC*H*₂CH₂Ar), 4.91 - 4.97 (m, 1H, C*H*CH₂Phenylpiperidin), 7.20 (t, J = 7.0 Hz, 1 H, Phenyl-C*H*, para), 7.25 - 7.38 (m, 5H, Phenyl-C*H*), 7.43 - 7.56 (m, 4H, Phenyl-C*H*), 7.60 (s, 1H, Pyrazol-C*H*).
**¹³C-NMR** (CDCl₃): δ (ppm) = 25.1 (1C, OCH₂*C*H₂Ar), 33.7 (2C, Phenylpiperidin-3',5'-CH₂), 42.9 (1C, Phenylpiperidin-4'-CH), 55.1, 55.6 (je 1C, Phenylpiperidin-2',6'-*C*H₂), 63.6 (1C, OCH₂CH₂Ar), 64.3 (1C, CH*C*H₂Phenylpiperidin), 71.3 (1C, *C*HCH₂Phenylpiperidin), 77.5 (1C, Pyrazol-4-C), 119.4 (1C, Phenylpiperidin-C, quartär), 112.9 (2C, Phenyl-CH, ortho), 126.4 (1C, Phenylpiperidin-CH, para), 127.1 (2C, Phenylpiperidin-CH, ortho), 127.2 (1C, Phenyl-CH, para), 128.7 (2C, Phenylpiperidin-CH, meta), 129.5 (2C, Phenyl-CH, meta), 136.1 (1C, Phenyl-C, quartär), 136.4 (1C, Pyrazol-3-CH), 139.8 (1C, Pyrazol-5-C).

### Example 3: 4-(Morpholin-4-ylmethyl)-1-phenyl-1,4,6,7-tetrahydro-pyrano[4,3-c]pyrazole

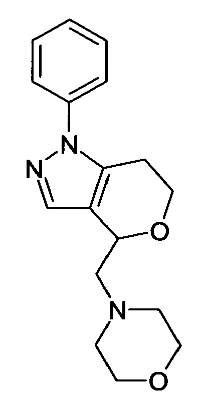

### Experimental Procedure:

To a solution of example B (100 mg, 0.34 mmol) in acetonitril (5 mL) were added consecutively K₂CO₃ (377 mg, 2.73 mmol) und morpholin (59.7 µL, 0.68 mmol). This reaction mixture was stirred for 47 h under reflux, before K₂CO₃ was filtered off and the solvent was removed in vacuum. The crude product (105 mg) was purified using flash-chromatography (∅ = 3 cm, h = 20 cm, n-hexane: ethylacetate 2:8, 20 mL, R_{f} = 0.06).
Colourless solid, melting point 115 °C, yield 59 mg (58%).
C₁₇H₂₁N₃O₂ (299.4)

| | C | H | N |
|---|---|---|---|
| Calc. | 68.2 | 7.07 | 14.0 |
| Found | 68.3 | 7.06 | 13.9 |

**MS** (ESI): m/z (rel. Int.) = 300 [MH⁺, 100], 621 [2 x M + Na⁺, 43].
**IR** (neat): *ṽ* (cm⁻¹) = 3061 (C-H _{aromat.}), 2967, 2918 (C-H _{aliphat.}), 2850 (C-H), 1599, 1505 (C=C), 1114, 1087 (C-O).
**¹H-NMR** (CDCl₃): δ (ppm) = 2.48 - 2.73 (m, 7H, Morpholin-2',6'-C*H₂* (4H), OCH₂*CH*₂Ar (1H), CHC*H₂*Morpholin (2H)), 2.94 - 3.05 (m, 1H, OCH₂C*H*₂Ar), 3.61 (ddd, J = 11.3/10.2/3.5 Hz, 1H, OC*H*₂C*H*₂Ar), 3.68 - 3.77 (m, 4H, Morpholin-3',5'-C*H*₂), 4.18 (ddd, J = 11.5/5.5/2.0 Hz, 1H, OC*H*₂CH₂Ar), 4.81 - 4.89 (m, 1H, C*H*CH₂Morpholin), 7.28 (t, J = 7.2 Hz, 1H, Phenyl-CH, para), 7.35 - 7.49 (m, 4H, Phenyl-*CH*), 7.51 (s, 1H, Pyrazol-3-*CH*).
**¹³C-NMR** (CDCl₃): δ (ppm) = 25.4 (1C, OCH₂*C*H₂Ar), 54.9 (2C, Morpholin-2',6'-*C*H₂), 64.0 (1C, OCH₂CH₂Ar), 64.5 (1C, CHCH₂Morpholin), 67.5 (2C, Morpholin-3',5'-*C*H₂), 71.2 (1C, CHCH₂Morpholin), 77.8 (1C, Pyrazol-4-C), 123.2 (2C, Phenyl-CH, ortho), 127.6 (1C, Phenyl-CH, para), 129.8 (2C, Phenyl-CH, meta), 136.4 (1C, Phenyl-C, quartär), 136.6 (1C, Pyrazol-3-CH), 140.0 (1C, Pyrazol-5-C).

### Example 4: 1-Phenyl-4-(4-phenylpiperazin-1-ylmethyl)-1,4,6,7-tetrahydropyrano[4,3-c]pyrazole

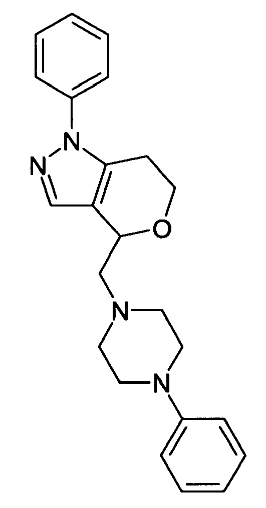

### Experimental Procedure:

To a solution of example B (80 mg, 0.27 mmol) in acetonitril (5 mL) were added consecutively K₂CO₃ (302 mg, 2.18 mmol) und phenylpiperazin (125 µL, 0.82 mmol). This reaction mixture was stirred for 41 h under reflux, before K₂CO₃ was filtered off and the solvent was removed in vacuum. The cruse product (135 mg) was prurified using flash-chromatography (∅=3 cm, h = 20 cm, n-hexane: ethylacetate 5:5 + 1% N,N-Dimethylethylamin, 20 mL, R_{f} = 0.18) and delivered the product.
Colourless Solid, Melting point 151 °C, Yield 68 mg (66%).
C₂₃H₂₆N₄O (374.5)

| | C | H | N |
|---|---|---|---|
| Calc. | 73.8 | 7.00 | 15.0 |
| Found | 73.6 | 7.08 | 14.6 |

**MS** (ESI): m/z (rel. Int.) = 375 [MH⁺, 100], 771 [2 x M + Na⁺, 43].
**IR** (neat): *ṽ* (cm⁻¹) = 3062 (C-H _{aromat.}), 2944 (C-H _{aliphat.}), 2852 (C-H), 1596, 1500 (C=C), 1098 (C-O).
**¹H-NMR** (CDCl₃): δ (ppm) = 2.63 - 2.84 (m, 7H, OCH₂C*H*₂Ar (1H), CHC*H*₂Phenylpiperazin (2H), Phenylpiperazin-3',5'-C*H*₂ (4H)), 3.01 - 3.12 (m, 1H, OCH₂C*H*₂Ar), 3.25 - 3.33 (m, 4H, Phenylpiperazin-2',6'-C*H*₂), 3.70 (ddd, J = 11.2/10.2/3.6 Hz, 1H, OC*H*₂CH₂Ar), 4.28 (ddd, J = 11.4/5.5/2.0 Hz, 1H, OC*H*₂CH₂Ar), 4.90 - 5.00 (m, 1H, C*H*-CH₂-Phenylpiperazin), 6.84 (t, J = 7.2 Hz, 1H, Phenylpiperazin-*C*H, para), 6.95 (d, J = 7.8 Hz, 2H, Phenylpiperazin-*C*H, ortho), 7.22 - 7.30 (m, 2H, Phenylpiperazin-*C*H, meta), 7.34 (t, J = 7.2 Hz, 1H, Phenyl-C*H*, para), 7.41 - 7.55 (m, 4H, Phenyl-*C*H), 7.59 (s, 1H, Pyrazol-3-C*H*).
**¹³C-NMR** (CDCl₃): δ (ppm) = 25.1 (1C, OCH₂CH₂Ar), 49.3 (2C, Phenylpiperazin-2',6'-CH₂), 54.2 (2C, Phenylpiperazin-3',5'-*C*H₂), 63.7 (1C, OCH₂CH₂Ar), 63.8 (1C, CHCH₂Phenylpiperazin), 71.2 (1C, CHCH₂Phenylpiperazin), 77.4 (1C, Pyrazol-4-C), 116.3 (2C, Phenylpiperazin-CH, ortho), 119.2 (1C, Phenylpiperazin-C, quartär), 119.9 (1C, Phenylpiperazin-CH, para), 122.9 (2C, Phenyl-CH, ortho), 127.3 (1C, Phenyl-CH, para), 129.4 (2C, Phenylpiperazin-CH, meta), 129.5 (2C, Phenyl-CH, meta), 136.1 (1C, Phenyl-C, quartär), 136.4 (1C, Pyrazol-3-CH), 139.7 (1C, Pyrazol-5-C).

### Example 5: 4-(4-Methylpiperazin-1-ylmethyl)-1-Phenyl-1,4,6,7-tetrahydropyrano[4.3-c]pyrazole

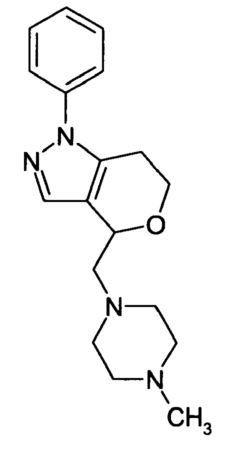

### Experimental Procedure:

To a solution of example B (100 mg, 0.34 mmol) in acetonitril (5 mL) were added consecutively K₂CO₃ (377 mg, 2.73 mmol) and 1-methylpiperazin (113.5 µL, 1.02 mmol). This reaction mixture was stirred for 42 h under reflux, before K₂CO₃ was filtered off and the solvent was removed in vacuum. The crude product (109 mg) was prurified using flash-chromatography (∅ = 3 cm, h = 18 cm, Ethylacetat + 5% N,N-Dimethylethylamin, R_{f} = 0.14) and delivered the product.
Colourless solid, melting point 66 °C, yield 42 mg (40%).
C₁₈H₂₄N₄O (312.5)

| | C | H | N |
|---|---|---|---|
| Calc. | 69.2 | 7.74 | 17.9 |
| Found | 69.2 | 7.79 | 17.4 |

MS (ESI): m/z (rel. Int.) = 313 [MH⁺, 100], 647 [2 x M + Na⁺, 13].
**IR** (neat): *ṽ* (cm⁻¹) = 2923 (C-H _{aliphat.}), 2851 (C-H), 1599, 1505 (C=C), 1093 (C-O), 759, 694 (C-H).
**¹H-NMR** (CDCl₃): δ (ppm) = 2.28 (s, 3H, Piperazin-C*H*₃), 2.43 - 2.75 (m, 11H, Methylpiperazin-2',3',5',6'-C*H*₂ (8H), CHC*H*₂Piperazin-CH₃ (2H), OCH₂C*H*₂Ar (1H)), 2.92 - 3.06 (m, 1H, OCH₂C*H*₂Ar), 3.61 (dd, J = 11.2/10.1/3.7 Hz, 1H, OC*H*₂CH₂Ar), 4.17 (ddd, J = 11.4/5.5/2.0 Hz, 1H, OC*H*₂CH₂Ar), 4.80 - 4.87 (m, 1H, C*H*CH₂-Piperazin-CH₃), 7.27 (t, J = 7.2 Hz, 1H, Phenyl-C*H*, para), 7.35 - 7.48 (m, 4H, Phenyl-C*H*), 7.50 (s, 1H, Pyrazol-3-C*H*).
**¹³C-NMR** (CDCl₃): δ (ppm) = 25.1 (1C, OCH₂*C*H₂Ar), 46.1 (1C, Piperazin-*C*H₃), 53.7, 55.1 (je 2C, Methylpiperazin-2',3',5',6'-*C*H₂), 63.56 (1C, O*C*H₂CH₂Ar), 63.59 (1C, CH*C*H₂Piperazin-CH₃), 71.2 (1C, *C*HCH₂Piperazin-CH₃), 77.5 (1C, Pyrazol-4-C), 122.9 (2C, Phenyl-CH, ortho), 127.3 (1C, Phenyl-CH, para), 129.5 (2C, Phenyl-CH, meta), 136.0 (1C, Phenyl-C, quartär), 136.4 (1C, Pyrazol-3-CH), 139.7 (1C, Pyrazol-5-C).

### Example 6: 1-Phenyl-4-(pyrrolidin-1-ylmethyl)-1,4,6,7-tetrahydro-pyrano[4,3-c]pyrazole

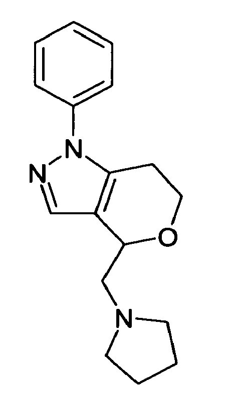

### Experimental Procedure:

To a solution of example B (100 mg, 0.34 mmol) in acetonitril (5 mL) were added consecutively K₂CO₃ (377 mg, 2.73 mmol) und pyrrolidin (56.0 µL, 0.68 mmol). This reaction mixture was stirred for 29 h under reflux, before K₂CO₃ was filtered off and the solvent was removed in vacuum. The crude product (94 mg) was purified using flash-chromatography (Ø=3 cm, h = 18 cm, n-hexane: ethylacetate 5:5 + 1% N,N-dimethylethylamine, 20 mL, R_{f} = 0.06) and delivered the product.
Colourless solid, melting point 89 °C, yield 46 mg (47%).
C₁₇H₂₁N₃O (283.4)

| | C | H | N |
|---|---|---|---|
| Calc. | 72.1 | 7.47 | 14.8 |
| Found | 71.6 | 7.49 | 14.5 |

**MS** (ESI): m/z (rel. Int.) = 284 [MH⁺, 100], 589 [2x M + Na⁺, 13].
**IR** (neat): *ṽ* (cm⁻¹) = 3061 (C-H _{aromat.}), 2961, 2929 (C-H _{allphat.}), 2854 (C-H), 1598, 1504 (C=C), 1090 (C-O).
**¹H-NMR** (CDCl₃): δ (ppm) = 1.72 - 1.83 (m, 4H, Pyrrolidin-3,4-C*H*₂), 2.55 - 2.64 (m, 5H, Pyrrolidin-2,5-C*H*₂ (4H), OCH₂C*H*₂Ar (1H)), 2.73 (dd, J = 12.7/3.3 Hz, 1H, CHC*H*₂Pyrrolidin), 2.82 (dd, J = 12.9/9.0 Hz, 1H, CHC*H*₂Pyrrolidin), 2.92 - 3.03 (m, 1H, OCH₂C*H*₂Ar), 3.61 (ddd, J = 11.2/10.3/3.7 Hz, 1H, OC*H*₂CH₂Ar), 4.09 (ddd, J = 11.4/5.6/2.3 Hz, 1H, OC*H*₂CH₂Ar), 4.77 - 4.84 (m, 1H, C*H*CH₂Pyrrolidin), 7.27 (t, J = 7.2 Hz, 1H, Phenyl-C*H*, para), 7.35 - 7.44 (m, 4H, Phenyl-C*H*), 7.46 (s, 1H, Pyrazol-3-CH).
**¹³C-NMR** (CDCl₃): δ (ppm) = 23.8 (2C, Pyrrolidin-3',4'-*C*H₂), 25.1 (1C, OCH₂*C*H₂Ar), 55.1 (2C, Pyrrolidin-2',5'-*C*H₂), 61.6 (1C, CHCH₂Pyrrolidin), 63.5 (1C, OCH₂CH₂Ar), 72.5 (1C, *C*HCH₂Pyrrolidin), 77.4 (1C, Pyrazol-4-C), 122.9 (2C, Phenyl-*C*H, ortho), 127.2 (1C, Phenyl-*C*H, para), 129.5 (2C, Phenyl-CH, meta), 136.1 (1C, Phenyl-C, quartär), 136.2 (1C, Pyrazol-3-CH), 139.8 (1C, Pyrazol-5-C).

### Example 7: N,N-Dimethyl-1-(1-phenyl-1,4,6,7-tetrahydropyrano[4,3-c]pyrazole-4-yl)methanamine

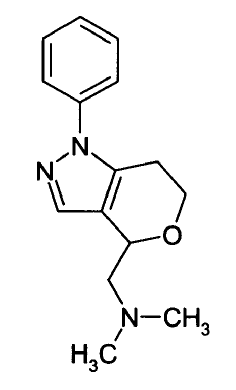

### Experimental Procedure:

To a solution of example B (100 mg, 0.34 mmol) in acetonitril (5 mL) were added consecutively K₂CO₃ (377 mg, 2.73 mmol) and - every 24 h - a solution of dimethylamine in THF (2 M, 1.7 mL, 3.40 mmol). This reaction mixture was stirred for 6 d under reflux, before K₂CO₃ was filtered of and the solvent was removed in vacuum. The crude product (95 mg) was purified using flash-chromatography (∅ = 3 cm, h = 18 cm, n-hexane: ethylacetate 1:1 + 2% N,N-dimethylethylamine, 20 mL, R_{f} = 0,08) and the product isolated.
Colourless solid, melting point 103 °C, yield 19 mg (22 %).
C₁₅H₁₉N₃O (257.4)

| | C | H | N |
|---|---|---|---|
| Calc. | 70.0 | 7.44 | 16.3 |
| Found | 70.2 | 7.62 | 15.6 |

**MS** (ESI): m/z (rel. Int.) = 258 [MH⁺, 100], 537 [2 x M + Na⁺, 5].
**IR** (neat): *ṽ* (cm⁻¹) = 3056 (C-H _{arcmat.}), 2972. 2952 (C-H ₐₗᵢₚₕₐₜ.), 2858, 2822 (C-H), 1599, 1505 (C=C), 1087 (C-O).
**¹H-NMR** (CDCl₃): δ (ppm) = 2.39 (s, 6H, N(C*H*₃)₂), 2.57 (dd, J = 13.3/3.5 Hz, 1H,CHC*H*₂N(CH₃)₂), 2.65 - 2.74 (m, 2H, CHC*H*₂N(CH₃)₂ (1H), OCH₂C*H*₂Ar (1H)), 3.02 - 3.13 (m, 1H, OCH₂C*H*₂Ar), 3.70 (ddd, J = 11.3/10.2/3.9 Hz, 1 H, OC*H*₂CH₂Ar), 4.25 (ddd, J = 11.4/5.7/2.2 Hz, 1H, OC*H*₂CH₂Ar), 4.80 - 4.88 (m, 1H, C*H*CH₂-N(CH₃)₂), 7.34 (t, J = 7.0 Hz, 1H, Phenyl-*C*H, para), 7.42 - 7.55 (m, 5H, Phenyl-*C*H (4H), Pyrazol-3-C*H* (1H)).
**¹³C-NMR** (CDCl₃): δ (ppm) = 25.1 (1C, OCH₂*C*H₂Ar), 46.4 (2C, N(*C*H₃)₂), 63.6 (1C, O*C*H₂CH₂Ar), 64.8 (1C, CH*C*H₂N(CH₃)₂), 71.5 (1C, *C*HCH₂N(CH₃)₂), 77.5 (1C, Pyrazol-4-C), 122.9 (2C, Phenyl-CH, ortho), 127.3 (1C, Phenyl-CH, para), 129.5 (2C, Phenyl-CH, meta), 136.08 (1C, Phenyl-C, quartar), 136.11 (1C, Pyrazol-3-CH), 139.8 (1C, Pyrazol-5-C).

In addition more compounds according to the invention are synthesized using the method of Reaction Scheme A in an analoguous way as in examples 1 to 7. This reaction and the resulting compounds (examples 8 to 14) are described in Scheme B: 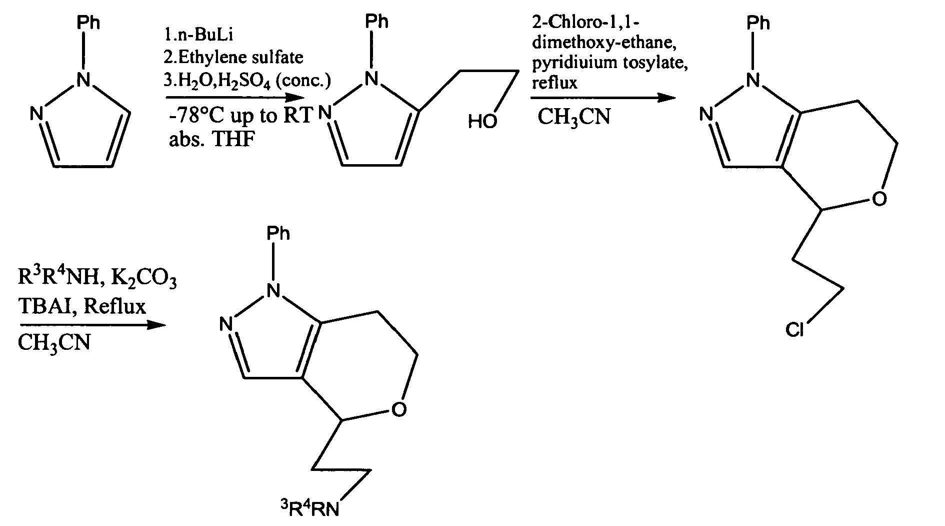 with R³ and R⁴ being:
- together piperidine;
- together 4-phenyl-piperidine;
- together morpholine;
- together 4-phenyl-piperazine;
- together 4-methyl-piperazine;
- together pyrolidine; or
- both methyl.

### BIOLOGICAL ACTIVITY

### A) In-Vitro

Some representative compounds of the invention were tested for their activity as sigma (sigma-1 and sigma-2) inhibitors. The following protocols were followed:

### Siama-1 (Version A)

Brain membrane preparation and binding assays for the σ1-receptor were performed as described (DeHaven-Hudkins et al., 1992) with some modifications. In brief, guinea pig brains were homogenized in 10 vols. (w/v) of Tris-HCl 50 mM 0.32 M sucrose, pH 7.4, with a Kinematica Polytron PT 3000 at 15000 r.p.m. for 30 s. The homogenate was centrifuged at 1000g for 10 min at 4°C and the supernatants collected and centrifuged again at 48000g for 15 min at 4°C. The pellet was resuspended in 10 volumes of Tris-HCl buffer (50 mM, pH 7.4), incubated at 37°C for 30 min, and centrifuged at 48000g for 20 min at 4°C. Following this, the pellet was resuspended in fresh Tris-HCl buffer (50 mM, pH 7.4) and stored on ice until use.
Each assay tube contained 10 µL of [³H](+)-pentazocine (final concentration of 0.5 nM), 900 µL of the tissue suspension to a final assay volume of 1 mL and a final tissue concentration of approximately 30 mg tissue net weight/mL. Non-specific binding was defined by addition of a final concentration of 1 µM haloperidol. All tubes were incubated at 37°C for 150 min before termination of the reaction by rapid filtration over Schleicher & Schuell GF 3362 glass fibre filters [previously soaked in a solution of 0,5% polyethylenimine for at least 1 h]. Filters were then washed with four times with 4 mL of cold Tris-HCI buffer (50 mM, pH 7.4). Following addition of scintillation cocktail, the samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

### Sigma 1 (Version B)

In brief the σ₁-receptor preparation was prepared from guinea pig brain. The brains were homogenized in 5 to 6 times of volume sucrose solution (0.32M) and homogenized. The homogenate was centrifuged at 2900 rpm, 4°C, 10 min). the supernatant was centrifuged again (23500 x g, 4°C, 20 min). The pellet was resuspended in Tris-buffer, incubated for 30 min at room temperature and centrifuged f (23500 x g, 4°C, 20 min). The pellet was resuspended in cold TRIS-buffer and homogenized. Then the protein content was measured (approx. 1.5 mg/mL) and the homogenate frozen at -80°C for later use.

The radioligand used was [³H]-(+)-Pentazocin in TRIS-buffer. In a volume of 200 µL 50 µL TRIS-buffer, 50 µL compound solution of varying concentration, 50 µL radioligand- solution (8 nM; resulting in 2 nM in the assay) and finally 50 µL of receptor preparation (approx. 1.5 mg /mL) were given into a well of a microplate equipped with a filter. The plate was closed and stirred for 2.5 h at 37 °C and 500 rpm. Following that the solvents were removed by a harvester through the filter. After rinsing with H₂O the filter was measured in a scintillation counter ([³H]-protocol).

### Sigma-2 (Version A)

Binding studies for σ2-receptor were performed as described (Radesca et al., 1991) with some modifications. In brief, brains from sigma receptor type I (σ1) knockout mice were homogenized in a volume of 10 mUg tissue net weight of ice-cold 10 mM Tris-HCI, pH 7.4, containing 320 mM sucrose (Tris-sucrose buffer) with a Potter-Elvehjem homogenizer (10 strokes at 500 r.p.m.) The homogenates were then centrifuged at 1000g for 10 min at 4°C, and the supernatants were saved. The pellets were resuspended by vortexing in 2 mUg ice-cold Tris-sucrose buffer and centrifuged again at 1000g for 10 min. The combined 1000g supernatants were centrifuged at 31000g for 15 min at 4°C. The pellets were resuspended by vortexing in 3 mUg 10 mM Tris-HCl, pH 7.4, and the suspension was kept at 25°C for 15 min. Following centrifugation at 31000g for 15 min, the pellets were resuspended by gentle Potter Elvehjem homogenization to a volume of 1.53 mUg in 10 mM Tris-HCl pH 7.4.

The assay tubes contained 10 µL of [³H]-DTG (final concentration of 3 nM), 400 µL of the tissue suspension (5.3 mUg in 50 mM Tris-HCl, pH 8.0) to a final assay volume of 0.5 mL. Non-specific binding was defined by addition of a final concentration of 1 µM haloperidol. All tubes were incubated at 25°C for 120 min before termination of the reaction by rapid filtration over Schleicher & Schuell GF 3362 glass fibre filters [previously soaked in a solution of 0,5% polyethylenimine for at least 1 h]. Filters were washed with three times with 5 mL volumes of cold Tris-HCI buffer (10 mM, pH 8.0). Following addition of scintillation cocktail samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

### References

DeHaven-Hudkins, D. L., L.C. Fleissner, and F. Y. Ford-Rice, 1992, "Characterization of the binding of [3H](+)pentazocine to σ recognition sites in guinea pig brain", Eur. J. Pharmacol. 227, 371-378.
Radesca, L., W.D. Bowen, and L. Di Paolo, B.R. de Costa, 1991, Synthesis and Receptor Binding of Enantiomeric N-Substituted cis-N-[2-(3,4-Dichlorophenyl)ethyl]-2-(1-pyrrolidinyl)cyclohexylamines as High-Affinity σ Receptor Ligands, J. Med. Chem. 34, 3065-3074.
Langa, F., Codony X., Tovar V., Lavado A., Giménez E., Cozar P., Cantero M., Dordal A., Hernández E., Pérez R., Monroy X., Zamanillo D., Guitart X., Montoliu LI., 2003, Generation and phenotypic analysis of sigma receptor type I (Sigma1) knockout mice, European Journal of Neuroscience, Vol. 18, 2188-2196.
Lowry, O.H., N.J. Rosebrough, A.L. Farr, and R.J. Randall, 1951, Protein measurement with the Folin phenol reagent, J. Biol. Chem, 193, 265.

### SIGMA 2 (Version B)

In brief the σ₂-Receptorpreparation was prepared from rat liver. The livers were homogenized in 5 to 6 times of volume sucrose solution (0.32M) and homogenized.

The homogenate was centrifuged at 2900 rpm, 4°C, 10 min). the supernatant was centrifuged again (31000 x g, 4°C, 20 min). The pellet was resuspended in TRIS-buffer, incubated for 30 min at room temperature while stirring and centrifuged f (31000 x g, 4°C, 20 min). The pellet was resuspended in cold TRIS-buffer pH 8 and homogenized. Then the protein content was measured (approx. 2 mg/mL) and the homogenate frozen at -80°C for later use.

The radioligand used was [³H]-Ditolylguanidin in TRIS-buffer pH 8. The σ₁-receptor binding sites were masked through (+)-Pentazocin-solution in TRIS-Puffer pH 8.

In a volume of 200 µL 50 µL compound solution of varying concentration, 50 µL (+)-Pentazocin- solution (2 µM; resulting in 500 nM in the assay), 50 µL radioligand-solution (12 nM; resulting in 3 nM in the assay) and finally 50 µL of receptor preparation (approx. 2 mg /mL) were given into a well of a microplate equipped with a filter. The plate was closed and stirred for 2 h at room temperature and 500 rpm. Following that the solvents were removed by a harvester through the filter. After rinsing with H₂O the filter was measured in a scintillation counter ([³H]-protocol).

Some of the results obtained (through the versions B) are shown in table (I).

**Table (I)**

| **Example** | **Binding σ1 Ki [nM]** | **Binding σ2 Ki [nM] or [Inhibition at 1µM]** |
|---|---|---|
| **1** | 10.5 ± 2.01 | 453 |
| **2** | 169 ± 69 | 5% |
| **3** | 42.3 ± 5.75 | 210 ± 94 |
| **4** | 219 ± 12.5 | 13% |
| **5** | 42.2 | 3070 |
| **6** | 1.60 ± 0.72 | 4.11 |
| **7** | 1380 | 12% |

### B) In-Vivo

### EFFECT ON CAPSAICIN IN DEVELOPMENT OF MECHANICAL ALLODYNIA

This model uses the von-Frey Filaments and is a model to test the effects or symptoms of neuropathic pain, allodynia etc.

Interest of the model:
- The injection of 1 µg of capsaicin to experimental animals produces acute pain followed by hyperalgesia/allodynia
- The mechanisms involved in capsaicin-induced acute pain and hyperalgesia are relatively well known (mainly activation of peripheral nociceptors and sensitization of spinal cord neurons, respectively)

The test protocol for all tests with of Frey filaments: After habituation mice were first treated with the test-compound (or solvent in controls). Then 1 µg capsaicin (1% DMSO) is injected into their paw resulting in developing pain in the effected paw. The effected paw is then treated with a mechanical stimulus and the latency time before the paw is withdrawn is measured.

## Claims

1. Compound of general formula (I), 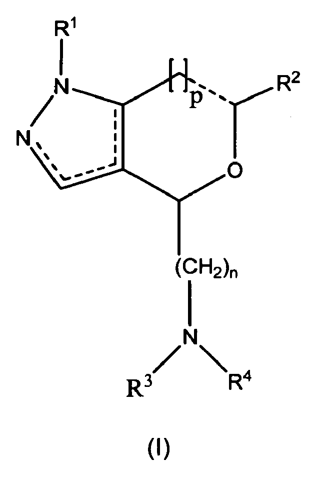 wherein
n is selected from 0,1, 2 or 3;
p is selected from 0 or 1;
the dotted line ......... is either a double or a single bond;
if p is 1, the dotted line ......... is either a double or a single bond;
if p is o, the dotted line ......... is a single bond;
R¹ is selected from hydrogen; at least mono-substituted, linear or branched C₁₋₆-aliphatic group; optionally at least monosubstituted aryl; optionally at least mono-subtituted alkyl-aryl;
R² is selected from hydrogen; an optionally at least monosubstituted, linear or branched C₁₋₆-aliphatic group; O-R with R being H or an optionally at least monosubstituted, linear or branched C₁₋₆-aliphatic group;
R³ and R⁴ independently of one another are selected from hydrogen; an optionally at least monosubstituted, linear or branched C₁₋₁₈-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;
or
R³ and R⁴ together with the connecting nitrogen form an optionally at least mono-substituted heterocyclyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

2. Compound according to claim 1, **characterized in that** R¹ is selected from hydrogen; at least mono-substituted, linear or branched C₁₋₆-aliphatic group; optionally at least monosubstituted aryl; especially R¹ is selected from linear or branched C₁₋₄-alkyl; or optionally at least monosubstituted aryl; more preferaby R¹ is selected from CH₃ or phenyl.

3. Compound according to any of claims 1 or 2, **characterized in that** R² is selected from H, or OR with R being H or an optionally at least monosubstituted, linear or branched C₁₋₆-aliphatic group; especially R² is selected from H or OR with R being H or a linear or branched C₁₋₄-alkyl group, more preferably R² is selected from H, OH or OCH₃; most preferably R² is selected from H.

4. Compound according to any of claims 1 to 3, **characterized in that**
R³ is selected from H; an optionally at least monosubstituted, linear or branched C₁₋₆-aliphatic group; an optionally at least monosubstituted alkyl-aryl; an optionally at least monosubstituted alkyl-heterocyclyl; or an optionally at least monosubstituted alkyl-cycloalkyl;
while R⁴ is selected from H; or an optionally at least monosubstituted, linear or branched C₁₋₆-aliphatic group;
or
R³ and R⁴ together with the connecting nitrogen form an optionally at least mono-substituted heterocyclyl group.

5. Compound according to any of claims 1 to 4, **characterized in that** n is selected from 1 or 2.

6. Compound according to claim 1, having a general formula la, 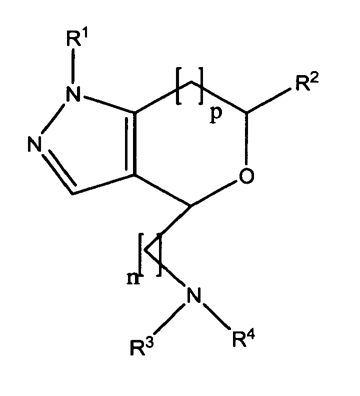 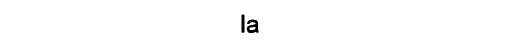 wherein
p is selected from 0 or 1;
n is selected from 1 or 2;
R¹ is selected from hydrogen; at least mono-substituted, linear or branched C₁₋₆-aliphatic group; optionally at least monosubstituted aryl; optionally at least mono-subtituted alkyl-aryl;
R² is selected from hydrogen; an optionally at least monosubstituted, linear or branched C₁₋₆-aliphatic group; or OR with R being H or an optionally at least monosubstituted, linear or branched C₁₋₆-aliphatic group;
R³ is selected from hydrogen; an optionally at least monosubstituted, linear or branched C₁₋₆-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;
while R⁴ is selected from hydrogen; an optionally at least monosubstituted, linear or branched C₁₋₆-aliphatic group;
or
R³ and R⁴ together with the connecting nitrogen form an optionally at least mono-substituted heterocyclyl group.

7. Compound according to claim 6, **characterized in that** R¹ is selected from hydrogen; at least mono-substituted, linear or branched C₁₋₆-aliphatic group; optionally at least monosubstituted aryl; especially R¹ is selected from linear or branched C₁₋₄-alkyl; or optionally at least monosubstituted aryl; more preferably R¹ is selected from CH₃ or phenyl.

8. Compound according to any of claims 6 or 7, **characterized in that** R² is selected from H; OR with R being H or an optionally at least monosubstituted, linear or branched C₁₋₆-alkyl group; especially R² is selected from H; OH or a linear or branched OC₁₋₄-alkyl group; more preferably R² is selected from H, OH or OCH₃.

9. Compound according to any of claims 6 to 8, **characterized in that** R³ is selected from hydrogen; an optionally at least monosubstituted, linear or branched C₁₋₆-alkyl;
while R⁴ is selected from hydrogen; or an optionally at least monosubstituted, linear or branched C₁₋₆-alkyl group;
or
R³ and R⁴ together with the connecting nitrogen form an optionally at least mono-substituted 5- or 6-membered saturated heterocyclyl group.

10. Compound according to claim 1 to 9, having a general formula la, 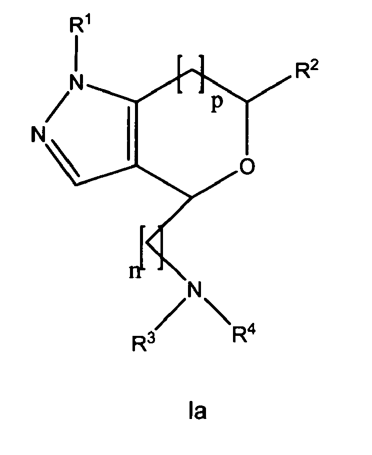 wherein
p is 1;
n is 1 or 2;
R¹ is selected from linear or branched C₁₋₄-alkyl; or optionally at least monosubstituted aryl;
R² is selected from H; OH or a linear or branched OC₁₋₄-alkyl group;
R³ is selected from hydrogen; an optionally at least monosubstituted, linear or branched C₁₋₆-alkyl;
while R⁴ is selected from hydrogen; or an optionally at least monosubstituted, linear or branched C₁₋₆-alkyl group;
or
R³ and R⁴ together with the connecting nitrogen form an optionally at least mono-substituted 5- or 6-membered saturated heterocyclyl group.

11. Compound according to claim 10, having a general formula la, 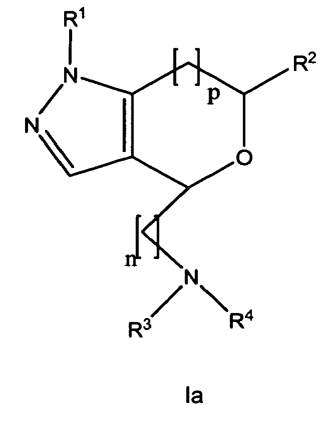 wherein
p is 1;
n is 1 or 2;
R¹ is selected from linear or branched C₁₋₄-alkyl; or optionally at least monosubstituted aryl;
R² is selected from H; OH or a linear or branched OC₁₋₄-alkyl group;
R³ is selected from hydrogen; an optionally at least monosubstituted, linear or branched C₁₋₆-alkyl;
while R⁴ is selected from hydrogen; or an optionally at least monosubstituted, linear or branched C₁₋₆-alkyl group;
or
R³ and R⁴ together with the connecting nitrogen form an optionally at least mono-substituted 5- or 6-membered saturated heterocyclyl group, selected from: 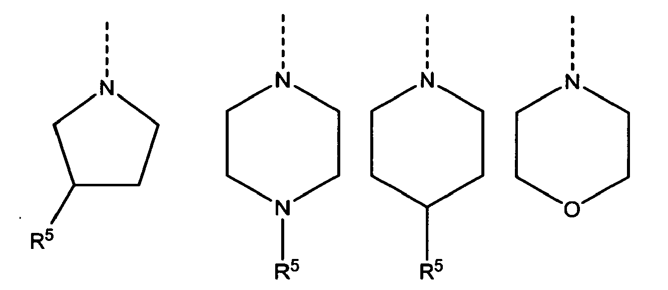
with R⁵ being selected from hydrogen; an optionally at least monosubstituted, linear or branched C₁₋₆-alkyl group; or an optionally at least monosubstituted aryl group.

12. Compound according to claim 1, **characterized in that** the compound is selected from
• 1-Phenyl-4-(piperidin-1-ylmethyl)-1,4,6,7-tetrahydro-pyrano[4,3-c]pyrazole;
• 1-Phenyl-4-(2-piperidin-1-yl-ethyl)-1,4,6,7-tetrahydro-pyrano[4,3-c]pyrazole
• 1-Phenyl-4-(4-phenylpiperidin-1-ylmethyl)-1,4,6,7-tetrahydropyrano[4,3-c]pyrazole;
• 1-Phenyl-4-[2-(4-phenyl-piperidin-1-yl)-ethyl]-1,4,6,7-tetrahydro-pyrano[4,3-c]pyrazole
• 4-(Morpholin-4-ylmethyl)-1-phenyl-1,4,6,7-tetrahydro-pyrano[4,3-c]pyrazole;
• 4-(2-Morpholin-4-yl-ethyl)-1-phenyl-1,4,6,7-tetrahydro-pyrano[4,3-c]pyrazole
• 1-Phenyl-4-(4-phenylpiperazin-1-ylmethyl)-1,4,6,7-tetrahydropyrano[4,3-c]pyrazole;
• 1-Phenyl-4-[2-(4-phenyl-piperazin-1-yl)-ethyl]-1,4,6,7-tetrahydro-pyrano[4,3-c]pyrazole;
• 4-(4-Methylpiperazin-1-ylmethyl)-1-phenyl-1,4,6,7-tetrahydropyrano[4,3-c]pyrazole;
• 4-[2-(4-Methyl-piperazin-1-yl)-ethyl]-1-phenyl-1,4,6,7-tetrahydro-pyrano[4,3-c]pyrazole
• 1-Phenyl-4-(pyrrolidin-1-ylmethyl)-1,4,6,7-tetrahydro-pyrano[4,3-c]pyrazole;
• 1-Phenyl-4-(2-pyrrolidin-1-yl-ethyl)-1,4,6,7-tetrahydro-pyrano[4,3-c]pyrazole;
• N,N-Dimethyl-1-(1-phenyl-1,4,6,7-tetrahydropyrano[4,3-c]pyrazole-4-yl)methanamine; or
• Dimethyl-[2-(1-phenyl-1,4,6,7-tetrahydro-pyrano[4,3-c]pyrazol-4-yl)-ethyl]-amine;
preferably
• 1-Phenyl-4-(piperidin-1-ylmethyl)-1,4,6,7-tetrahydro-pyrano[4,3-c]pyrazole;
• 1-Phenyl-4-(4-phenylpiperidin-1-ylmethyl)-1,4,6, 7-tetrahydropyrano[4,3-c]pyrazole;
• 4-(Morpholin-4-ylmethyl)-1-phenyl-1,4,6,7-tetrahydro-pyrano[4,3-c]pyrazole;
• 1-Phenyt-4-(4-phenylpiperazin-1-ylmethyl)-1,4,6,7-tetrahydropyrano[4,3-c]pyrazole;
• 4-(4-Methylpiperazin-1-ylmethyl)-1-phenyl-1,4,6,7-tetrahydropyrano[4,3-c]pyrazole;
• 1-Phenyl-4-(pyrrolidin-1-ylmethyl)-1,4,6,7-tetrahydro-pyrano[4,3-c]pyrazole; or
• N,N-Dimethyl-1-(1-phenyl-1,4,6,7-tetrahydropyrano[4,3-c]pyrazole-4-yl)methanamine;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

13. Process for the production of a compound according to formula lb, 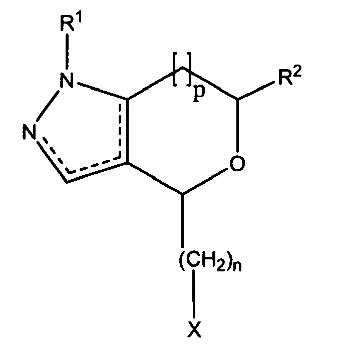 wherein R¹, R² n, and p are as defined in claim 1 and X is a leaving group, preferably a halogen,
wherein a compound of formula III 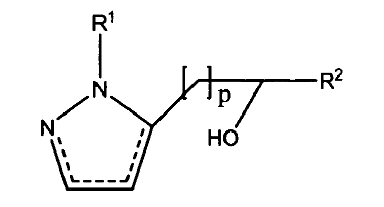 , wherein R¹, R² and p are as defined in claim 1, is reacted with a compound of general formula IV 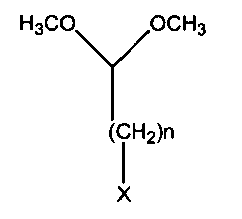 wherein n is as defined in claim 1 and X is a leaving group, preferably is a halogen, more preferably is Br or Cl;
to form a compound according to formula lb.

14. Process for the production of a compound according to claim 1, wherein a compound of formula lb, 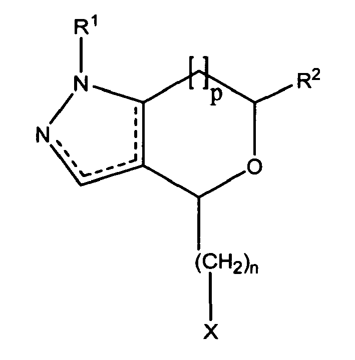 wherein R¹, R² n and p are as defined in claim 1 and X is a leaving group, preferably a halogen, is reacted with R³R⁴NH, wherein R³ and R⁴ are as defined in claim 1.

15. A pharmaceutical composition which comprises a compound as defined in any of claims 1-12 or a pharmaceutically acceptable salt, prodrug, isomer or solvate thereof, and a pharmaceutically acceptable carrier, adjuvant or vehicle.

16. Use of a compound as defined in any of claims 1-12 in the manufacture of a medicament.

17. Use of a compound as defined in any of claims 1-12 in the manufacture of a medicament for the treatment or prophylaxis of a sigma receptor mediated disease or condition.

18. Use according to claim 17 wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer.

19. Use according to claim 17 wherein the disease is pain, especially neuropathic pain, inflammatory pain or other pain conditions, allodynia and/or hyperalgesia, especially mechanical allodynia.

20. Use of a compound as defined in any of claims 1-12 as pharmacological tool or as anxiolytic or immunosuppressant.
